(19) Europäisches Patentamt
European Patent Office
Office européen des brevets

(11) **EP 4 781 912 A1**

(12) # EUROPEAN PATENT APPLICATION

(43) Date of publication:
**29.07.2026 Bulletin 2026/31**

(21) Application number: **26153182.6**

(22) Date of filing: **21.01.2026**

(51) International Patent Classification (IPC):
**A61B 5/287** (2021.01)   **A61B 5/341** (2021.01)
**A61B 5/367** (2021.01)

(52) Cooperative Patent Classification (CPC):
**A61B 5/287; A61B 5/341; A61B 5/367;**
A61B 2017/00243; A61B 2034/2051;
A61B 2034/2053

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH LA MA MD TN**

(30) Priority: **22.01.2025 US 202519033798**

(71) Applicant: **Biosense Webster (Israel) Ltd.
2066717 Yokneam (IL)**

(72) Inventor: **STEIN, Asaf
2066717 Yokneam (IL)**

(74) Representative: **Carpmaels & Ransford LLP
One Southampton Row
London WC1B 5HA (GB)**

(54) **PACING MAPPING OF VENTRICULAR TACHYCARDIA (VT) WITH DIRECTION VECTOR INDICATION**

(57)    A system includes a catheter, an electrocardiogram (ECG) system, and a processor. The catheter is configured to apply pacing to given tissue location inside a ventricle of heart of patient. The ECG system is configured to acquire a set of ECG signals generated in response to the pacing, wherein electrodes of the ECG system attached to the patient define respective directions in space relative to an origin located in the heart. The processor is configured to (i) calculate respective set of signal differences between the acquired set of ECG signals and reference set of ECG signals, (ii) calculate corresponding set of deviation vectors along the respective directions in space, the deviation vectors having amplitudes corresponding to the respective signal differences, (iii) using the deviation vectors, calculate direction vector from the given location to new ventricle location at which to apply the pacing, and (iv) indicate calculated direction vector to user.

**EP 4 781 912 A1**

## Description

### FIELD OF THE DISCLOSURE

This disclosure relates generally to electrophysiological (EP) signals, and specifically to evaluation of electrical propagation in the heart.

### BACKGROUND OF THE DISCLOSURE

Estimation of electrophysiological signals to determine the location of ventricular arrhythmia was previously suggested in patent literature. For example, U.S. Patent Application Publication 2024/0374199 describes a method that includes receiving cardiac signals from multiple locations within the ventricle of the heart of a patient. The received signals are compared with reference signals indicative of an arrhythmia. Based on the comparison, a direction is calculated towards a location that may demonstrate an increased correlation between the received signals and the reference signals. The direction is indicated to a user.

The present disclosure will be more fully understood from the following detailed description of the examples thereof, taken together with the drawings, in which:

### BRIEF DESCRIPTION OF THE DRAWINGS

Fig. 1 is a schematic, pictorial illustration of a catheter-based electrophysiology (EP) pacing, mapping, and ablation system, according to an example of the present disclosure;

Figs. 2A and 2B are the transverse and sagittal views of the 12-ECG electrodes of Fig. 1 orientations, respectively, relative to a location inside the heart, according to an example of the present disclosure;

Fig. 3 is a graph of reference and acquisition sets of EP signals from the 12-ECG electrodes of Fig. 1, according to an example of the present disclosure;

Fig. 4 is a schematic, pictorial illustration of a layout of deviation vectors derived from pacing data acquired by the system of Fig. 1 to guide a user to a left ventricle (LV) arrhythmogenic region, according to an example of the present disclosure; and

Fig. 5 is a flow chart describing a method to guide a clinician to an LV arrhythmogenic location using data acquired by an ECG system, according to an example of the present disclosure.

### DETAILED DESCRIPTION OF EXAMPLES

OVERVIEW

To characterize an arrhythmia of a cardiac chamber, such as a left ventricle (LV), a physician may use a catheter to pace (e.g., apply bipolar pacing signals between two adjacent catheter electrodes) at multiple LV tissue locations in search of suspected tissue pathways and circuits within the LV. During the pacing, if a transient arrhythmogenic event is induced, such as an ectopic beat, premature beat, or premature ventricle complex (PVC), the event may be recorded with a 12-lead ECG device showing an abnormal signal pattern.

Various methods can be used to recognize the induced arrhythmogenic event at a given ventricle location. For example, a processor may perform pattern matching (e.g., of 12-lead ECG waveforms) between acquired waveforms and stored pattern waveforms characteristic of the arrhythmia (e.g., VT, PVC) to identify correlations. A high correlation indicates that the paced location is part of an arrhythmogenic tissue.

It is often difficult for a physician to determine in which direction to move the catheter in order to obtain a meaningfully high correlation. Therefore, the traditional process of acquiring good-quality data and finding meaningful correlations involves attempted pacing at multiple tissue locations, a workflow that can be difficult to fulfill in practical clinical scenarios.

Some examples of the present disclosure that are described hereinafter provide a technique to calculate and display a direction arrow on a cardiac map (e.g., an EP map) after one or more pacing instances to direct the operator toward the best location for the next pacing and/or to the arrhythmogenic tissue region. The size of the displayed arrow indicates the distance from the current paced location and the next best pacing location and/or the arrhythmogenic tissue region.

The direction arrow in 3D space is directly derived from a scaled difference vector in 3D space extracted using a mathematical calculation to relate 12-lead ECG acquired waveforms to reference 12-lead ECG waveforms characteristic of the arrhythmia. The disclosed technique provides a simple workflow for a clinician to identify a VT target location with a minimal number of pacing steps during the clinical procedure. This workflow significantly increases the efficiency and accuracy of VT pacing mapping.

In one example, a method is provided that includes sending a pacing signal to a catheter and receiving, in response, ECG signals from a 12-lead recorder. A correlation level is calculated between the received ECG signals and ECG reference signals indicative of an arrhythmia. A set of amplitude-deviation vectors in 3D space of given amplitudes (e.g., given in mV) is calculated between each received ECG signal and the respective reference ECG signal (e.g., at the peak of the received ECG signal). The direction of each amplitude-deviation vector is set along each respective ECG lead's directionality relative to a location in the heart.

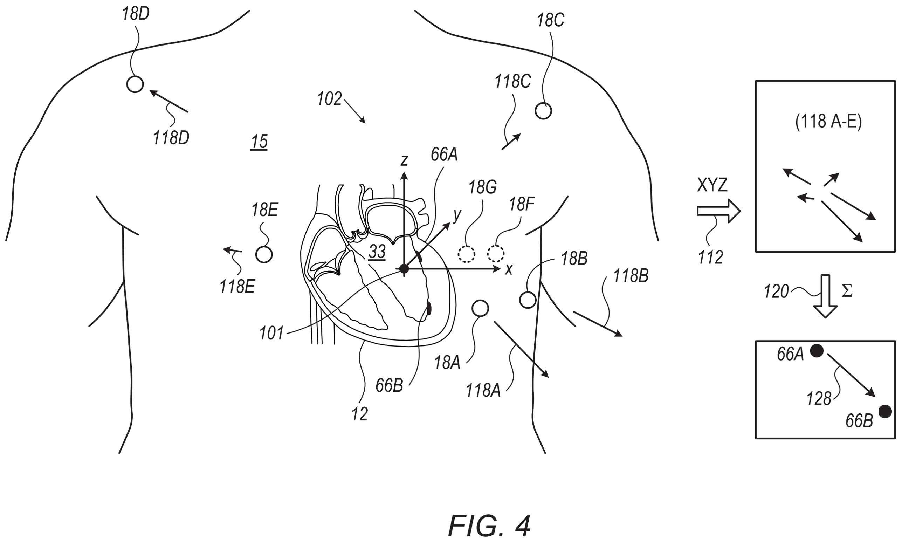

Summing over all amplitude-deviation vectors (as seen in Fig. 4) gives the difference vector. The resulting difference vector points in the correct direction of the VT focus, or, at least, toward the next ventricle location recommended for pacing. The direction is represented on the cardiac map by an arrow that directs the operator where to adjust the catheter location for the next pacing. The size of the displayed arrow depends on the magnitude of the difference vector (a larger size indicates that larger catheter movement is required in the designated direction).

The arrow is refreshed with additional pacing steps until the arrow is small enough to conclude (e.g., graphically indicate on the map) that the target region has been reached and/or that the calculated correlation is sufficiently high (e.g., above a predefined threshold).

In another example, a time difference (e.g., in mSec) is calculated between each current ECG signal and the reference ECG signal (e.g., between a peak of the current ECG signal and that of the reference signal). This time difference also serves as the magnitude of a 3D vector with a directionality between a given origin location in the heart and each ECG lead's location. The difference vector is determined by summing over all time-difference vectors. The resulting difference vector also points in the correct direction of the VT focus.

In yet another example, the difference vector is calculated by a weighted summation over all the amplitude-deviation vectors or time-difference vectors. The weights are deduced either by a model, or empirically, to reflect the 12-lead geometry and cardiac anatomy to improve the accuracy of the difference vector and, henceforth, the guiding arrow presented to the clinician.

SYSTEM DESCRIPTION

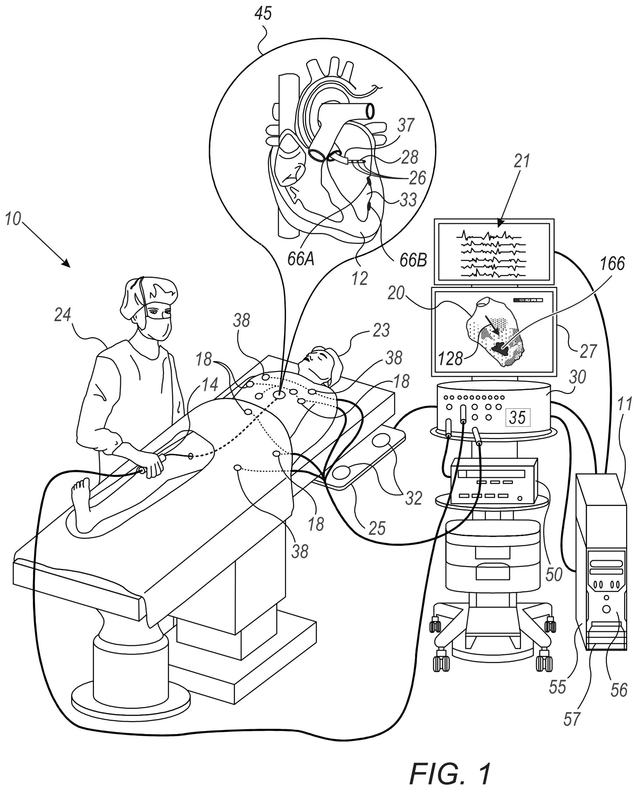

Fig. 1 is a schematic, pictorial illustration of a catheter-based electrophysiology (EP) pacing, mapping, and ablation system 10, according to an example of the present disclosure.

System 10 includes a catheter 14 which is percutaneously inserted via a sheath by physician 24 through the patient's vascular system into a left ventricle (LV) 33 of heart 12 of a patient 23. Catheter 14, illustrated herein, is configured for unipolar or bipolar pacing. Physician 24 brings a tip distal end assembly 28 of catheter 14 into contact with the heart wall for pacing locations over a given area of LV 33.

As seen in inset 45, tip distal end assembly 28 carries several electrodes 26 for pacing and optionally for electrically ablating LV 33 wall tissue that is found to be arrhythmogenic.

Catheter 14 may further carry a magnetic position sensor that is operated together with a location pad 25 that includes a plurality of magnetic coils 32 configured to generate magnetic fields in a predefined working volume. The real-time position of distal tip 28 of catheter 14 may be tracked based on magnetic fields generated with location pad 25 and sensed by the magnetic position sensor. Details of the magnetic position sensing technology are described in U.S. Patent Nos. 5,5391,199; 5,443,489; 5,558,091; 6,172,499; 6,239,724;

6,332,089; 6,484,118; 6,618,612; 6,690,963; 6,788,967; 6,892,091.

System 10 includes one or more electrode patches 38 positioned for skin contact on patient 23 to establish a location reference for location pad 25 as well as impedance-based tracking of electrodes 26. For impedance-based tracking, electrical current is directed toward electrodes 26 and sensed at electrode skin patches 38 so that the location of each electrode can be triangulated via electrode patches 38. Details of the impedance-based location tracking technology are described in US Patent Nos. 7,536,218; 7,756,576; 7,848,787; 7,869,865; and 8,456,182.

A recorder 11 displays cardiac signals 21 (e.g., electrograms from a 12-lead ECG device 35 acquired with body surface ECG electrodes 18). Recorder 11 may include pacing capability to pace the heart rhythm and/or may be electrically connected to a standalone pacer.

Patient interface unit (PIU) 30 is an interface configured to establish electrical communication between catheters, electrophysiological equipment, power supply and a workstation 55 to control system 10 operation and receive EP signals from the catheter or apply pacing signals. Electrophysiological equipment of system 10 may include, for example, multiple catheters, location pad 25, body surface ECG electrodes 18, electrode patches 38, an ablation energy generator 50, and recorder 11. Optionally, and preferably, PIU 30 additionally includes processing capability for implementing real-time computations of catheter locations and for performing ECG calculations.

Workstation 55 includes memory 57, a processor 56 unit with memory or with appropriate operating software loaded therein, and user interface capability. Workstation 55 may provide multiple functions, optionally including (i) modeling endocardial anatomy in three-dimensions (3D) and rendering the model or anatomical map 20 for display on a display device 27, (ii) displaying on display device 27 activation sequences (or other data) compiled from recorded cardiac signals 21 in representative visual indicia or imagery superimposed on the rendered anatomical map 20, (iii) displaying real-time location and orientation of multiple catheters within the heart chamber, and (iv) displaying sites of interest on display device 27, such as places where ablation energy has been applied. One commercial product embodying elements of system 10 is available as the CARTO™ 3 System, available from Biosense Webster, Inc., 31A Technology Drive, Irvine, CA 92618.

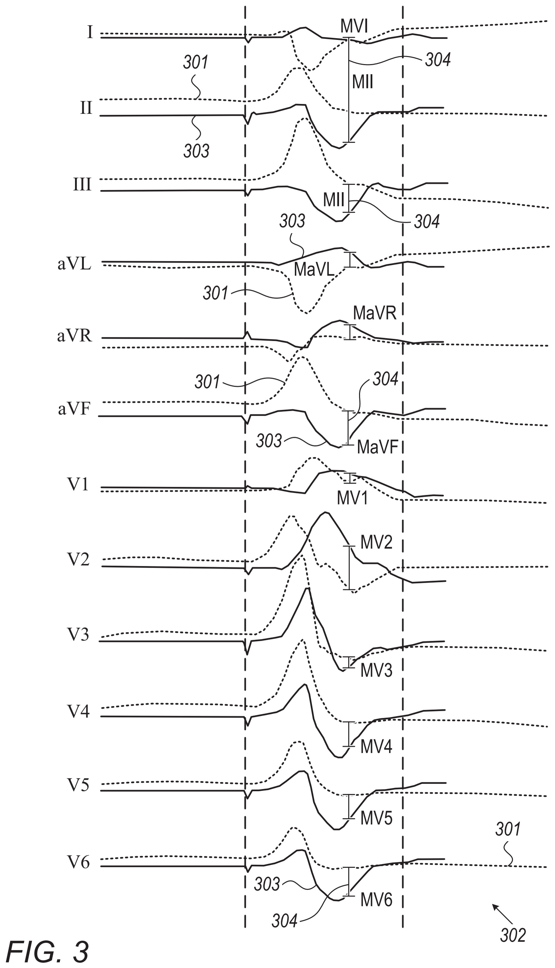

In Fig. 1, catheter 14 paces LV tissue location 66A. The resulting ECG signal correlation with the reference signal is found to be too low, meaning that location 66A is not VT arrhythmogenic tissue, i.e., the correlation alone is not sufficient for the physician to proceed with pacing. Using the disclosed technique, a direction vector 128 is calculated from the sets of ECG signals acquired at location 66A and the reference signal (e.g., signals of Fig. 3). Scaled direction vector 128, overlayed on map 20, guides clinician 24 (e.g., physician 24) to move catheter 14 in the correct direction. In the shown example, vector 128 points toward LV tissue location 66B that should be more arrhythmogenic (and therefore yield a higher correlation indication (166)). It is also possible that location 66B actually is the target location, in which case physician 24 may ablate the region about location 66B.

In some examples, processor 56 typically comprises a general-purpose computer, which is programmed in software to carry out the functions described herein. The software may be downloaded to the computer in electronic form, over a network, for example, or it may, alternatively or additionally, be provided and/or stored on non-transitory tangible media, such as magnetic, optical, or electronic memory.

System 10 may include an ablation energy generator 50 that is adapted to conduct ablative energy to one or more electrodes at a distal tip of a catheter configured for ablation. Energy produced by ablation energy generator 50 may include, but is not limited to, radiofrequency (RF) energy or pulsed-field ablation (PFA) energy, including monopolar or bipolar high-voltage DC pulses, to be used to effect irreversible electroporation (IRE), or combinations thereof.

For ablation, physician 24 similarly brings a distal end of an ablation catheter to a target site. One or more additional catheters can be inserted via the sheath. They may include a catheter for sensing intracardiac electrogram signals, a catheter dedicated for ablating and/or a catheter dedicated for both EP mapping and ablating.

This configuration of system 10 is shown by way of example, in order to illustrate certain problems that are addressed by examples of the present disclosure and to demonstrate the application of these examples in enhancing the performance of such a system. Examples of the present disclosure, however, are by no means limited to this specific sort of example system, and the principles described herein may similarly be applied to other sorts of medical systems. For example, other catheter types may be used, such as the LASSO™ catheter or a basket catheter.

PACING MAPPING OF VT WITH DIRECTION VECTOR INDICATION

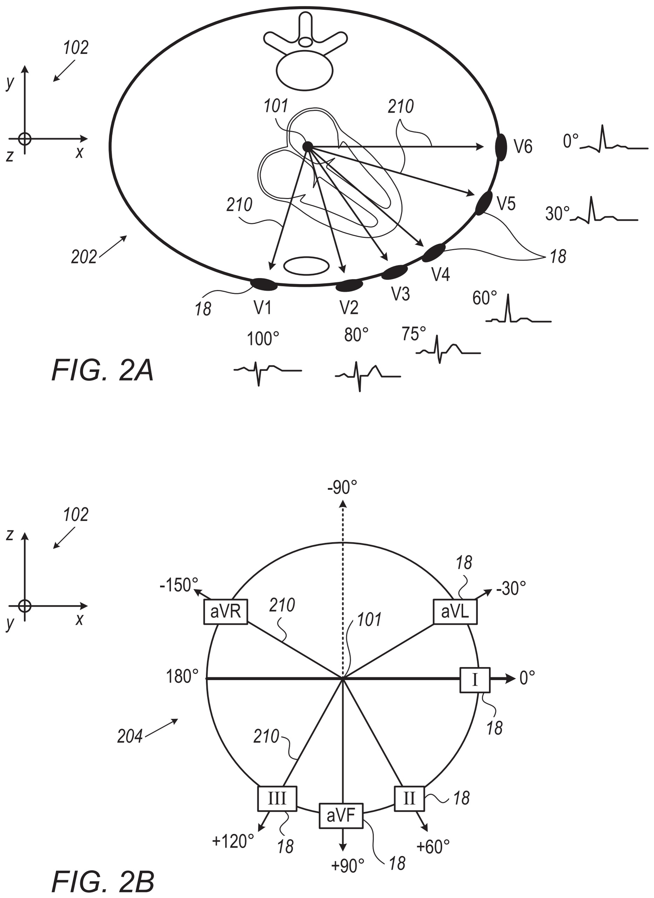

Figs. 2A and 2B are the transverse 202 and sagittal 204 views of the 12-ECG electrodes 18 of Fig. 1 orientations 210, respectively, relative to a location 101 inside heart 12, according to an example of the present disclosure. The transverse and sagittal planes define x-y and x-z planes, respectively, of an XYZ system 102 of mutually orthogonal axes (also shown in Fig. 3). Directions of ECG electrodes disposed over the patient torso are defined in system 102 relative to the location origin 101.

As seen in Fig 2, the 12 axes of the 12-ECG leads, while not designed to span the entire 3D space, are nevertheless useful in spanning a direction inside a ventricle, such as the direction of vector 128 of Fig. 1. This spanning is derived by projecting ECG signal differences on directions 210, as described in Fig. 4. The technique thus obtains 12 deviation vectors 118 of Fig. 4 to derive from the aforementioned scaled difference vector 128, which is then presented as a pacing guiding arrow on cardiac map 20 of the ventricle.

Fig. 3 is a graph 302 of reference set 301, acquired during a VT event, and acquisition 303 sets of EP signals from the 12-ECG electrodes 18 of Fig. 1, according to an example of the present disclosure. Using the very same set of electrodes 18 attached to the patient's skin, set 303 is acquired during the diagnostic phase described in Fig. 1 (pacing). The difference in line shapes between sets 301 and 303 reflects the fact that set 303 was obtained when pacing a location that is removed from the arrhythmogenic tissue region (e.g., normal tissue was paced).

The disclosed technique quantifies the differences in signals in the form of amplitudes 304 (e.g., given in mV). These amplitudes may be viewed as quantifying how different the acquired signal is from the respective reference signal at a given electrode 18. As seen, some of the amplitudes 304 are small while others are large. Amplitudes 304 are used in Fig. 4, below, to calculate a respective set of amplitude-deviation vectors 118 in 3D space.

Fig. 4 is a schematic, pictorial illustration of a layout of deviation vectors 118 derived from pacing data acquired by the system of Fig. 1 to guide a user to a left ventricle (LV) arrhythmogenic region, according to an example of the present disclosure. Fig. 4 shows some ECG electrodes 18, where electrodes 18A-E are located at the frontal side (e.g., chest 15) of a patient, and electrodes 18F-G are located on the patient's side or back.

As noted above, direction 210 can be defined in system 102 of XYZ axes from origin 101 to each electrode 18. Using amplitudes 304 of Fig. 3, the processor calculates (112) amplitude-deviation vectors 118A-E (shown by way of example, where in practice, all 12 vectors are calculated). Weighted summation (120) of amplitude-deviation vectors 118A-E gives a scaled direction vector $\mathbf{V}$, 128,

$$V = C \sum w_i M_i \widehat{u_i} \quad ,$$

where $\mathbf{w_i}$ is a weight of electrode $18_i$, i=1, 2..., 12, $M_i$ is the respective magnitude 304, and $\widehat{u_i}$ 210 is the direction from origin 101 to each electrode $18_i$. $M_i \widehat{u_i}$ is difference vector 118(i), and $\mathbf{C}$ is a scaling coefficient described below. It was found by the inventor that using $\mathbf{w_i = 1}$ for all weights yields a sufficiently accurate direction vector $\mathbf{V}$, 128. However, putting another weight $\mathbf{w_i}$ to

electrode 18 may further improve accuracy.

The scaling coefficient *C* of vector 128 of the units [cm/mV] enables presenting vector 128 as an arrow on map 20 between the current pacing location 66A and the next recommended pacing location 66B. The typical size of the scaling coefficient *C* falls within a range of 0.5-2 [cm/mV]. The arrow is presented on cardiac map 20 to guide physician 24.

METHOD OF PACING MAPPING OF VT WITH DIRECTION VECTOR INDICATION

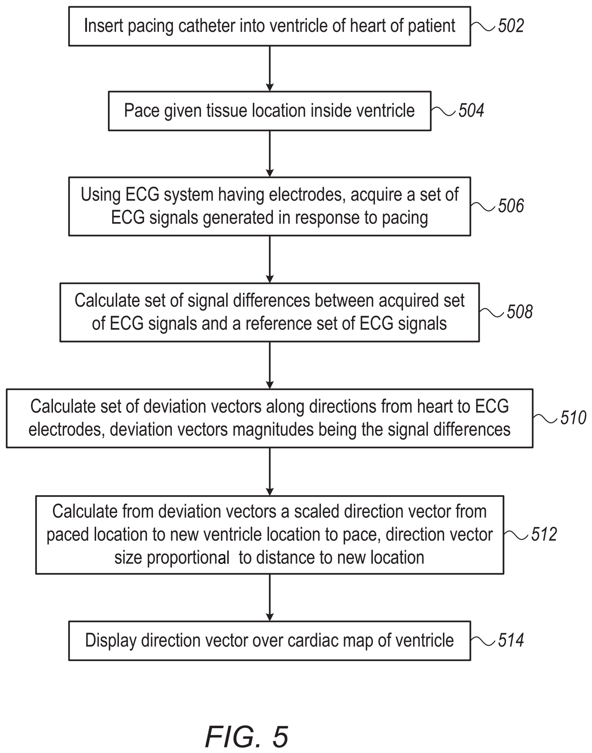

Fig. 5 is a flow chart describing a method to guide a clinician toward an LV arrhythmogenic location using data acquired by ECG system 11, according to an example of the present disclosure. As described above, each electrode 18 of the ECG system 11 attached to the patient defines a direction 210 in space relative to an origin 101 located in the heart.

The algorithm, according to the presented example, carries out a process that begins with inserting a pacing catheter 14 into ventricle 33 of heart 12 at catheter insertion step 502.

Next, using catheter 14 electrodes, system 10 paces a given tissue location inside the ventricle, at a pacing step 504.

In response to the pacing, at a signal receiving step 506, system 10 receives respective ECG signals from body surface electrodes 18.

Next, at amplitudes calculation step 508, processor 56 calculates a respective set of signal differences 304 between the acquired set 303 of ECG signals and a reference 301 set of ECG signals. The processor plugs the amplitudes (i.e, the measured signals minus the respective reference signals) into a vector equation of the algorithm.

At deviation vectors calculation step 510, processor 56 calculates a corresponding set of deviation vectors 118 along the respective directions 210 in space, the deviation vectors having the calculated amplitudes 304.

At direction vector calculation step 512, the processor uses the deviation vectors to calculate a scaled direction vector 128 from the given location 66A to a new ventricle location 66B to pace.

The processor displays the calculated scaled direction vector 128 over a cardiac map 20 of at least a portion of the ventricle, at arrow presentation step 514.

The flowchart of Fig. 5 is brought by way of example. For example, additional steps may be included, such as uploading from memory 57 a set of weights, $w_i$, and scaling coefficient C, to use in calculating direction vector 128, which are omitted for simplicity.

EXAMPLES

Example 1

A system (10) includes a catheter (14), an electrocardiogram (ECG) system (35), and a processor (56). The catheter is configured to apply pacing to given tissue location inside a ventricle of heart (12) of patient (23). The ECG system is configured to acquire a set of ECG signals (303) generated in response to the pacing, wherein electrodes (18) of the ECG system (35) attached to the patient define respective directions (210) in space relative to an origin (101) located in the heart. The processor is configured to (i) calculate respective set of signal differences (304) between the acquired set of ECG signals (303) and reference set of ECG signals (301), (ii) calculate corresponding set of deviation vectors (118) along the respective directions (210) in space, the deviation vectors having amplitudes corresponding to the respective signal differences (304), (iii) using the deviation vectors (118), calculate a direction vector (128) from the given location (66A) to new ventricle location (66B) at which to apply the pacing, and (iv) indicate calculated direction vector (128) to user.

Example 2

The system (10) according to claim 1, wherein the processor (56) is configured to calculate the direction vector (128) by performing a weighted summation over the deviation vectors (118).

Example 3

The system (10) according to claim 1, wherein the signal differences (304) are one of amplitude differences and time differences.

Example 4

The system (10) according to claim 1, wherein the ECG system (35) is a 12-lead ECG system.

Example 5

The system (10) according to claim 1, wherein the origin (101) is one of (i) a sinus node (SA) and (ii) the given tissue location (66A) at which the pacing is applied.

Example 6

The system (10) according to claim 1, wherein the acquired set of ECG signals (303) and the reference set of ECG signals (301) are obtained using the same electrodes (18) of the ECG system (35).

Example 7

The system (10) according to claim 1, wherein the processor (56) is further configured to indicate the calculated direction vector (128) to a user by displaying the calculated direction vector (128) over a cardiac map (20) of at least a portion of the ventricle.

Example 8

The system (10) according to claim 1, wherein the processor (56) is further configured to indicate the ventricle new location (66B) as an arrhythmogenic location if a size of the direction vector (128) is smaller than a predefined size.

Example 9

A method includes applying pacing to a given tissue location inside a ventricle of a heart (12) of a patient using a catheter (14). A set of ECG signals (303) generated in response to the pacing is acquired using an electrocardiogram (ECG) system (35), wherein electrodes (18) of the ECG system attached to the patient define respective directions (210) in space relative to an origin (101) located in the heart. A respective set of signal differences (304) is calculated between the acquired set of ECG signals (303) and a reference set of ECG signals (301). A corresponding set of deviation vectors (118) along the respective directions (210) in space is calculated, the deviation vectors having amplitudes corresponding to the respective signal differences. A direction vector (128) is calculated using the deviation vectors, from the given location (66A) to a new ventricle location (66B) at which to apply the pacing. The calculated direction vector (128) is indicated to a user.

It will be appreciated that the examples described above are cited by way of example, and that the present disclosure is not limited to what has been particularly shown and described hereinabove. Rather, the scope of the present disclosure includes both combinations and subcombinations of the various features described hereinabove, as well as variations and modifications thereof which would occur to persons skilled in the art upon reading the foregoing description and which are not disclosed in the prior art.

**Claims**

1. A system (10), comprising:

   a catheter (14) configured to apply pacing to a given tissue location inside a ventricle of a heart (12) of a patient (23); an electrocardiogram (ECG) system (35) configured to acquire a set of ECG signals (303) generated in response to the pacing, wherein electrodes (18) of the ECG system (35) attached to the patient define respective directions (210) in space relative to an origin (101) located in the heart; and

   a processor (56), which is configured to:

      calculate a respective set of signal differences (304) between the acquired set of ECG signals (303) and a reference set of ECG signals (301); calculate a corresponding set of deviation vectors (118) along the respective directions (210) in space, the deviation vectors having amplitudes corresponding to the respective signal differences (304); using the deviation vectors (118), calculate a direction vector (128) from the given location (66A) to a new ventricle location (66B) at which to apply the pacing; and indicate the calculated direction vector (128) to a user.

2. The system (10) according to claim 1, wherein the processor (56) is configured to calculate the direction vector (128) by performing a weighted summation over the deviation vectors (118).

3. The system (10) according to any of claims 1 and 2, wherein the signal differences (304) are one of amplitude differences and time differences.

4. The system (10) according to any of claims 1 through 3, wherein the ECG system (35) is a 12-lead ECG system.

5. The system (10) according to any of claims 1 through 4, wherein the origin (101) is one of (i) a sinus node (SA) and (ii) the given tissue location (66A) at which the pacing is applied.

6. The system (10) according to any of claims 1 through 5, wherein the acquired set of ECG signals (303) and the reference set of ECG signals (301) are obtained using the same electrodes (18) of the ECG system (35).

7. The system (10) according to any of claims 1 through 6, wherein the processor (56) is further configured to indicate the calculated direction vector (128) to a user by displaying the calculated direction vector over a cardiac map (20) of at least a portion of the ventricle.

8. The system (10) according to any of claims 1 through 7, wherein the processor (56) is further configured to indicate the ventricle new location (66B) as an arrhythmogenic location if a size of the direction vector (128) is smaller than a predefined size.

9. A method, comprising:

   applying pacing to a given tissue location inside a ventricle of a heart of a patient using a catheter (14);

acquiring a set of ECG signals (303) generated in response to the pacing using an electrocardiogram (ECG) system (35), wherein electrodes (18) of the ECG system attached to the patient (23) define respective directions (210) in space relative to an origin (101) located in the heart;

calculating a respective set of signal differences (304) between the acquired set of ECG signals (303) and a reference set of ECG signals (301);

calculating a corresponding set of deviation vectors (118) along the respective directions (210) in space, the deviation vectors having amplitudes corresponding to the respective signal differences (304);

using the deviation vectors (118), calculating a direction vector (128) from the given location (66A) to a new ventricle location (66B) at which to apply the pacing; and

indicating the calculated direction vector (128) to a user.

FIG. 1

FIG. 2A

FIG. 2B

FIG. 3

FIG. 4

EP 4 781 912 A1

Insert pacing catheter into ventricle of heart of patient — *502*

Pace given tissue location inside ventricle — *504*

Using ECG system having electrodes, acquire a set of ECG signals generated in response to pacing — *506*

Calculate set of signal differences between acquired set of ECG signals and a reference set of ECG signals — *508*

Calculate set of deviation vectors along directions from heart to ECG electrodes, deviation vectors magnitudes being the signal differences — *510*

Calculate from deviation vectors a scaled direction vector from paced location to new ventricle location to pace, direction vector size proportional to distance to new location — *512*

Display direction vector over cardiac map of ventricle — *514*

## FIG. 5

## EUROPEAN SEARCH REPORT

**Application Number**

EP 26 15 3182

Europäisches Patentamt
European Patent Office
Office européen des brevets

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2021/113345 A1 (UNIV JOHNS HOPKINS [US]) 10 June 2021 (2021-06-10) | 1-7,9 | INV.<br>A61B5/287 |
| Y | * paragraphs [0023] - [0030], [0041]; figures 2,8 * | 8 | A61B5/341<br>A61B5/367 |
| X | US 2016/089046 A1 (KOLANDAIVELU ARAVINDAN [US]) 31 March 2016 (2016-03-31) | 1,4,9 | |
| Y | * paragraphs [0018] - [0025], [0032]; figures 3,4 * | 8 | |
| A | US 5 391 199 A (BEN-HAIM SHLOMO [IL]) 21 February 1995 (1995-02-21)<br>* column 5, line 32 - column 8, line 68 *<br>* column 11, line 10 - column 11, line 42 *<br>* column 12, line 41 - column 12, line 65 *<br>* figures 2,3,7 * | 1-9 | |

**TECHNICAL FIELDS SEARCHED (IPC)**

A61B

The present search report has been drawn up for all claims

| Place of search | Date of completion of the search | Examiner |
|---|---|---|
| Berlin | 4 May 2026 | Schatz, Veronika |

## ANNEX TO THE EUROPEAN SEARCH REPORT
## ON EUROPEAN PATENT APPLICATION NO.

EP 26 15 3182

This annex lists the patent family members relating to the patent documents cited in the above-mentioned European search report.
The members are as contained in the European Patent Office EDP file on
The European Patent Office is in no way liable for these particulars which are merely given for the purpose of information.

04-05-2026

| Patent document cited in search report | | | Publication date | Patent family member(s) | | Publication date |
|---|---|---|---|---|---|---|
| WO 2021113345 | A1 | | 10-06-2021 | US | 2023011001 A1 | 12-01-2023 |
| | | | | WO | 2021113345 A1 | 10-06-2021 |
| US 2016089046 | A1 | | 31-03-2016 | US | 2016089046 A1 | 31-03-2016 |
| | | | | WO | 2014190119 A1 | 27-11-2014 |
| US 5391199 | A | | 21-02-1995 | AT | E290818 T1 | 15-04-2005 |
| | | | | AU | 692789 B2 | 18-06-1998 |
| | | | | CA | 2144946 A1 | 02-02-1995 |
| | | | | DE | 69434296 T2 | 08-06-2006 |
| | | | | DK | 0679068 T3 | 11-07-2005 |
| | | | | EP | 0679068 A1 | 02-11-1995 |
| | | | | ES | 2240964 T3 | 16-10-2005 |
| | | | | JP | 4040672 B2 | 30-01-2008 |
| | | | | JP | H08504653 A | 21-05-1996 |
| | | | | PT | 679068 E | 29-07-2005 |
| | | | | US | 5391199 A | 21-02-1995 |
| | | | | US | 5443489 A | 22-08-1995 |
| | | | | US | 5480422 A | 02-01-1996 |
| | | | | US | 5546951 A | 20-08-1996 |
| | | | | US | 5568809 A | 29-10-1996 |
| | | | | US | 5694945 A | 09-12-1997 |
| | | | | US | 5713946 A | 03-02-1998 |
| | | | | US | 5840025 A | 24-11-1998 |
| | | | | WO | 9502995 A1 | 02-02-1995 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20240374199
- US 55391199 B
- US 5443489 A
- US 5558091 A
- US 6172499 B
- US 6239724 B
- US 6332089 B
- US 6484118 B
- US 6618612 B

- US 6690963 B
- US 6788967 B
- US 6892091 B
- US 7536218 B
- US 7756576 B
- US 7848787 B
- US 7869865 B
- US 8456182 B